# EUROPEAN PATENT APPLICATION

(11) **EP 4 477 173 A1**
(43) Date of publication of application: **18.12.2024**
(21) Application number: 23315247.9
(22) Date of filing: 15.06.2023
(51) Int. Cl.: A61B 34/10, A61B 34/00, A61B 34/30, A61B 34/32, A61B 34/35

(54) **SYSTEM FOR AUTONOMOUSLY CARRYING OUT A MEDICAL PROCEDURE**

(71) Applicant: Caranx Medical, 75013 Paris (FR)
(72) Inventor: MOSCU, Mircea, 06600 Antibes (FR); PERARD, Luc, 06700 Saint-Laurent-Du-Var (FR); PRZYBYLSKI, Flavie, 06800 Cagnes sur Mer (FR); BERTHET-RAYNE, Pierre, 06800 Vagnes-sur Mer (FR); CERRUTI, Giulio, 06700 Saint-Laurent-Du-Var (FR); MÎRA, Anna, 06000 Nice (FR); SMITS, Jonas Victor Harmen, 3360 Bierbeek (FR)
(74) Representative: Hepp Wenger Ryffel AG

(57) **Abstract**

The invention relates to a system (1), am method and a computer program for autonomously carrying out a medical procedure comprising at least one step. The system (1) comprises a computing unit (3), an interface (4) for communication with least one actuator (5) for operating a surgical robot (6) and a user interface (2) for entering user instructions. The computing unit (3) is adapted to autonomously control operation of the actuator (5) to carry out the at least one step of the medical procedure, to receive control instructions from said user interface (2) in the form of one of change instructions, stop instructions and continue instructions and to induce a change operation, a stop operation or a continue operation of the actuator based on the control instructions.

## Description

The present invention relates to a system, a method and a computer program for autonomously carrying out a medical procedure according to the independent claims.

Robotic surgery or robot-assisted surgery allows doctors to perform many types of complex procedures with more precision, flexibility and control than it is possible with conventional techniques. Robotic surgery is usually associated with minimally invasive surgery, procedures performed through tiny incisions. It is also sometimes used in certain traditional open surgical procedures.

The most widely used clinical surgical robots include a camera arm and mechanical arms with surgical instruments attached to them.

As shown in "Autonomy in Surgical Robots", Annual Review of Control, Robotics, and Autonomous Systems, Volume 4, 2021, Attanasio, pp 651-679, autonomy achievable by a surgical robot system can be classified into six levels, based on an initial proposal by Yang et al. (2017, Medical robotics-regulatory, ethical, and legal considerations for increasing levels of autonomy, Sci. Robot., 2:eaam8638) and inspired by the taxonomy and definition of the levels of autonomy by the SAE J3016 standard (2), which defines similar levels for on-road autonomous vehicles.

The six levels are defined as follows: level 0 (no autonomy, wherein the motion of the robot is controlled exclusively by the operator, with no supports or constraints provided), level 1 (robot assistance, wherein the system provides active constraints to guide the operator's motion or virtual fixtures to enhance the surgical site visualization), level 2 (task autonomy, wherein the system is capable of accomplishing specific surgical tasks based on specifications provided by the operator), level 3 (conditional autonomy, wherein the system understands the surgical scenario, plans and executes specific tasks, and updates the plan during execution), level 4 (high autonomy, wherein the system interprets preoperative and intraoperative information, devises an interventional plan composed of a sequence of tasks, executes this plan autonomously, and replans if necessary), and level 5 (full autonomy).

While autonomous driving has reached level 3 (conditional autonomy) and is approaching level 4 (high autonomy), the commercially available platforms for robotic surgery remain solidly at level 0 (no autonomy).

Robotic assisted surgery of level 0 for example is known for mitral valve repair, thoracoscopic surgery and robotic-assisted surgery.

In the context of surgical robotics, ethical and legal concerns arise regarding the consequences of decision errors and incorrect robot behaviours, potentially leading to serious injuries or even death.

It is an object of the present invention to overcome the drawbacks of the prior art. In particular, the system and the method according to the present invention shall provide robotic surgery which satisfy the clinical needs and, in particular the respective applicable legal requirements and which allows a high level of autonomy.

These and other objects are solved with a system, a method and a computer program according to the independent claims.

The invention provides a system for autonomously carrying out a medical procedure comprising at least one step.

The system comprises a computing unit, an actuator interface for communication with at least one actuator for operating at least one surgical robot and a user interface for entering user instructions.

Within this application a user generally is a human user.

The actuator is a component that converts electrical signals into mechanical motion or other physical actions, e.g. increase of pressure, change of temperature etc., and that is responsible for moving or controlling a mechanism or system of the surgical robot, for example by moving a component or opening a valve.

Within this application a surgical robot may comprise at least one robot arm, at which a light source, a sensor device and/or a surgical device is attached or attachable. The sensor device may for example comprise a camera or an ultrasound probe. The surgical device may comprise surgical tools, such as a guidewire, an endoscope, a puncturing needle, a deployment tool comprising an implant valve or a dilatation balloon.

The computing unit is adapted to autonomously control operation of the actuator to carry out at least one of the at least one step of the medical procedure.

The computing unit may also be adapted to perform a user-guided operation of the actuator to carry out a step of the medical procedure.

The computing unit is further adapted to receive control instructions from said user interface in the form of one of change instructions, stop instructions and continue instructions.

A change instruction may serve for selecting, defining and/or amending surgical steps to be operated by the surgical robot.

The change instruction may be a new instruction, which does not refer to a pending or a running surgical step.

A stop instruction serves for finishing or interrupting a surgical step or a sequence of surgical steps.

A continue instruction serves for validating or confirming a surgical step or a sequence of surgical steps.

The computing unit is further adapted to induce a change operation, warning signal, a to-proceed signal, a stop operation or a continue operation of the actuator based on the control instructions.

The computing unit may be adapted to generate a signal based on the control instructions and to send the signal to the actuator interface.

The computer unit may be adapted to plan and/or execute at least one step of the medical procedure. For planning and executing steps the computer unit may be adapted to use AI (artificial intelligence) software.

The computing unit may provide an autopilot function for executing at least one step of a medical procedure.

The computing unit may be adapted to simulate surgical steps, and, in particular, to determine critical phases and critical areas.

The computing unit may be adapted to determine next steps of the medical procedure and to generate output data which provide information about possible subsequent steps and/or their implication.

The computing unit may be adapted to compute the outcome of user input/user alterations and to determine possible risks associated with the respective input.

The computing unit may be adapted to assist a surgical step fully controlled by a user, for example to emit sound and/or light signals during the surgical step, for example during navigation, to help with localisation, or to supress tremor movements of the operator.

The computing unit may be adapted to validate a surgical step, for example to validate that an implant is correctly placed and anchored by an operator.

The computing unit may be adapted to log steps of the medical procedure, and interactions with an operator/user.

The actuator interface may comprise a socket or a plug for cooperating with a plug or a socket of a surgical robot. The actuator interface may comprise a wireless connection for cooperating with the surgical robot.

The user interface may be a master interface for communication with medical staff. The user interface may comprise at least one of a monitor, a touchscreen, a joystick, a handle, a control panel, a motion tracker, a gaze tracker, a microphone and a voice recognizer, a speaker, a camera and an image recognizer.

The medical procedure may be divided into a plurality of separated steps.

The system may be adapted to automatically request or to receive said control instructions before, after and/or during each of the steps.

For example, the system may be adapted to automatically request or to receive said control instructions during a step in case of an emergency, detected by the system, by the user or by an external system.

Alternatively, the system may be adapted to automatically request to receive said control instructions before and/or after a sequence of steps.

Depending on a predetermined criticality of each of the steps the system may be adapted to automatically request to receive control instructions only before steps which are more critical.

The system may also be adapted to automatically request to receive said control instructions during a step, for example when a single step takes a longer time, such as a navigation step.

The computing unit may be adapted to receive an emergency stop instruction at any time.

An operator may be asked by the system to validate each step or to validate only individual steps. An operator may interact at each step.

The computing unit may be operable in an autonomous mode, in a fully degraded mode and in partially degraded modes.

In the autonomous mode, the computing unit fully controls the operation of the actuator and eventually of non-mechanical operations.

In the autonomous mode, the system enacts each step without human validation. The system acts fully on inputs other than human. The autonomous mode corresponds to level 5 (full autonomy) as described above.

In the fully degraded mode, the computing unit either releases the actuator so that it can be freely and manually operated by a user in a manual degraded mode or the computing unit controls the actuator based on teleoperation control instructions provided by a user in a teleoperated degraded mode.

In the manual degraded mode the systems operates fully manual. The operator can directly physically interact with the surgical robot.

Alternatively, the surgical robot may be operated in a teleoperated degraded mode, wherein the actuator is controlled by the computing unit receiving teleoperation control instructions from a teleoperation user interface. The operator can directly control the surgical robot via the computing unit.

In the fully degraded mode, the computing unit may only provide output, i.e., send control signals to the actuator interface, based on human input. The computing unit does not perform any decision-making processes and leaves complete control to the operator. However, there might be some algorithmic autonomy in the fully degraded mode, such as tremor suppression and redundancy resolution, which do not interfere with the operator's actions.

The fully degraded mode corresponds to the level 0 as discussed above.

In a partially degraded mode the computing unit controls the actuator to carry out at least one step of the medical procedure based on communication with a user.

Among the partially degraded modes a distinction can be made between different levels of the partially degraded modes.

The system may operate autonomously with supervision. A user may give go/no-go inputs on each predefined step. The system may act fully on inputs other than human, except for validation of intermediate steps. The level corresponds to level 4 (high autonomy) as described above, wherein the computing unit interprets preoperative and intraoperative information, devises an interventional plan composed of a sequence of tasks, induces execution of this plan autonomously and replans if necessary. An operator supervises the system under the discrete control paradigm.

In the partially degraded mode the system may operate in a degraded manner, but augmented corresponding to levels 1, 2 and/or 3 as described above.

In level 1 the system may provide some support to the operator/user, but may never take control of the action being performed. The system may support the operator in the execution of a specific surgical action. The system may for example provide passive assistance by providing additional information to the operator, when asked for. The system may provide an enhanced imaging, for example augmented reality. The system may provide active assistance systems, for example in restricting the motion of surgical instruments.

In level 2 the computing unit may control steps of procedure but is not allowed to define any parameter for planning a task. The operator provides the information required to perform the action.

In level 3, the system may conceive strategies to perform a specific task, while always relying on a human operator to approve the most suitable strategy to be implemented. Parts of the procedure may be performed autonomously such as navigation of flexible endoscopic robots in unstructured environments or the localization of an access point for an intraluminal operation.

In the fully and partially degraded modes the system may receive direct input via a GUI (e.g. for access point selection for an intraluminal entrance) and/or other input systems (e.g. a gamepad for setting a needle depth or a position and orientation of an ultrasound probe).

In the fully and partially degraded modes in case a step is not validated, the system may await input from a human which can be a retry command or a direct order (e.g., if an AI-determined access point is incorrect, the user can specify via the GUI where the actual access point should be).

The system may comprise a switch unit for switching operation between the autonomous mode, the fully degraded mode and/or the partially degraded modes.

Alternatively or additionally, the system may comprise a switch unit for switching operation between different levels of the partially degraded mode.

Preferably, the operation mode is selected before the first step of the medical procedure. However, the switch unit may be adapted for switching operation on demand at any time during the medical procedure. Thus, an operator may alter the level of autonomy corresponding to the course of the surgical procedure. When the operator considers it to be necessary, he or she may take more control, or he or she may release control to the system.

In the autonomous mode and in the partially degraded modes, steps of the surgical procedure may run automatically until they are stopped or until a stop criterium occurs.

An operator may be forced by the system to supervise the automatically running steps, for example by a dead man's switch.

The switch unit may be controlled by the computing unit in such a way that operation can be automatically switched from the autonomous mode to the fully or partially degraded mode and operation can only be switched to the autonomous mode upon instruction or validation by a user.

In a similar manner, the operation may be automatically switched to a lower level of autonomy whereas for a change to a higher level of autonomy instruction and/or validation of a user is necessary.

Generally, the computing unit may be operated in a pyramid structure of decision taking. The lowest level is associated with a low level signal processing, the next level uses AI (artificial intelligence) for image and/or sensor processing, the next level uses AI for context interpretation and in the highest level, all steps of the procedure may be controlled by the computing unit.

Context sensitive AI may provide for a downgrade of the level of autonomy or a stop of the procedure but may not upgrade the level of autonomy.

However, the computing unit gives full power to the operator at any time of the procedure for any level of autonomy, if requested by the operator.

The computing unit may be adapted to receive a switch instruction from said user interface and/or to induce a switch based on the switch instruction, in particular to change the level of autonomy to a higher level of autonomy.

The system may comprise a validation interface for entering validation instructions. The validation interface preferably is selected from the group of a vocal interface, a mechanical button or switch, a mouse, joystick, haptic glove, a graphical user interface, in particular a touchscreen, a motion detector, in particular a gaze tracker or a head motion detector, a virtual reality or an augmented reality interface, an eye-to-image interface, a 3D monitor, in particular a hologram presentation combined with a haptic glove or a motion detection, and a timer.

The validation interface may be an interface for a user input. The user interface may comprise the validation interface.

Alternatively or additionally, the validation interface may receive non-human input, for example from a timer or a sensor device.

The computing unit may be adapted to compare a signal received by the validation interface from an external device, such as a timer or a sensor, with a predetermined condition, and to provide a signal or an action based on the comparison.

The system may comprise a change interface for entering change instructions, as well as initiate instructions, proceed instructions, hold instructions or stop instructions. The change interface is preferably selected from the group of a vocal interface, a mechanical button or switch, a mouse, joystick, haptic glove, a graphical user interface, in particular a touchscreen, a motion detector, in particular a gaze tracker or a head motion detector, an eye-to-image interface, a virtual reality or an augmented reality interface, and a 3D monitor, in particular a hologram presentation combined with a haptic glove or a motion detector.

The change interface is an interface for a user input to the system, in particular to the computing unit, and the user interface may comprise the change interface.

Preferably the system is suitable for use in an intraluminal procedure, in particular for an intravascular or gastrointestinal surgical procedure, such as placing and fixing an implant within a body lumen of a patient.

The change instructions may be selected from the group of defining an access point for entering the patient's body, defining a position of the surgical robot with respect to an access point for entering the patient's body, defining an implant position, defining a speed and a trajectory of an implant insertion, defining a target position and/or orientation of an implant, defining deployment parameters for deploying an implant, in particular a balloon pressure for expanding an implant, defining an orientation and a speed of instruments during instrument retraction and operation of an imaging device, in particular an endoscopic camera, and defining a checklist and/or a to-do list comprising steps of the intervention.

A checklist comprising steps of the intervention may be defined in a machine-readable format and/or may be defined in a format for outputting the list on an output interface, such that a user may check, modify and/or update the list.

Within this context "defining" means, that either all information concerning the respective action is entered by the user or the computing unit is adapted to define the information needed for the respective action on demand or the computing unit is adapted to amend a predetermined action.

The change instruction may comprise the full information concerning the respective action, i.e. a respective action is fully defined by information entered by the user, or the change instruction may comprise a command relating to the respective action, i.e. the computer unit determines the information needed for the respective action.

The computing unit may define the respective action based on data entered by the user, based on data of a database stored in the system or in a memory and/or based on data provided by a further system, for example a sensor device.

For example, for defining an access point for entering the patient's body the user may enter all data for a chosen target site on the patient's body, or the computing unit may be asked to calculate an access point, for example via the change interface, for example based on an image analysis. The respective change instruction may concern the change of a previously entered or previously defined access point.

For example, for defining the position of the surgical robot with respect to an access point for entering the patient's body, the user may enter all data necessary for placing the surgical robot, for example directly place the surgical robot with a chosen orientation at a chosen site via the change interface. In this case the computing unit is operated based on the change instruction entered by the user.

Alternatively, the respective change instructions may concern starting a positioning routine where the computing unit may define the path and the orientation of the surgical robot and/or may control the movement of the surgical robot autonomously.

Alternatively, the change instruction may concern the change of a previously entered or previously calculated position and orientation of the surgical robot.

Analogously, for defining an implant position, for example of a heart valve, the user may enter all data for the final implant site, for example via the change interface, or the computing unit may adapted to define the implant site and the user may enter a command for defining the implant position.

Alternatively, the respective change instruction concerns the change of a previously entered or previously determined implant position.

Analogously, for defining a speed and a trajectory of an implant insertion, in particular for intravascular placing of a heart valve, the user may enter all data for the speed and the trajectory of an implant insertion, for example via the change interface. Alternatively, the computing unit is adapted to define the speed and the trajectory of an implant insertion and may be prompted to determine the speed and the trajectory.

Alternatively, the respective change instruction may concern the change of a previously entered or previously determined speed and trajectory of an implant insertion.

Analogously, for defining a detailed position and/or an orientation of an implant the user may enter all data for the detailed position and/or the orientation of the implant, for example via the change interface, or the computing unit may be adapted to define the detailed position and/or the orientation of the implant and the user may enter a command for prompting the computer unit to define the detailed position and/or the orientation.

Alternatively, the respective change instruction may concern the change of a previously defined detailed position and/or orientation of the implant.

Analogously, for defining deployment parameters for deploying an implant the user may enter all data of the deployment parameters, for example via the change interface, or the computing unit may be asked to determine the deployment parameters. Alternatively, the respective change instruction concerns the change of a previously entered or previously determined deployment parameters.

Analogously, for defining an orientation and a speed of instruments during instrument retraction the user may enter all data of orientation and the speed of instruments, for example via the change interface, or the computing unit may be adapted to define the orientation and the speed of instruments and the user may enter a command to induce calculation of the orientation and the speed. Alternatively, the respective change instruction may concern the change of a previously defined orientation and speed of instruments.

For example, for defining a checklist comprising all steps of the intervention, all steps or a part of the steps may be manually entered by a user, and/or steps may be proposed by the system and/or a checklist may be edited during planning and/or a checklist may be updated either automatically or manually.

The system may further comprise a stop interface for entering stop instructions. The stop interface preferably is selected from the group of a vocal interface, a mechanical button or switch, a mouse, joystick, haptic glove, a graphical user interface, in particular a touchscreen, a motion detector, in particular a gaze tracker or a head motion detector, a virtual reality or an augmented reality interface, a 3D monitor, in particular a hologram presentation combined with a haptic glove or a motion detection, a timer, a button and a detection device for detection of a risk or an unexpected event.

The detection device may be a device for detection of environmental conditions, a device for detection of the system status or a device for detection of patient characteristics, such as vital signs of the patient. The detection device may provide a stop instruction in case a signal captured by the detection device is below or above a predefined threshold.

The detection device may provide a stop instruction in case a failure of a system component is detected or in case the patient behaves unexpectedly, for example starts moving.

The stop instruction may provide for the computing unit to immediately stop a surgical step or for downgrading the level of autonomy.

The system may have a memory for storing a risk level associated with at least one of said steps.

The risk levels may be prestored. The operator may assign certain predetermined risk levels to respective steps and/or the operator may specify certain risk levels. The operator may change the risk level for an ongoing procedure step.

The operator may change the risk level for at least one step before the medical procedure or during the medical procedure, but before the step is carried out.

A certain risk level may be associated with a respective predetermined instruction criterion, for example a respective input by a user.

A certain risk level may be associated with a certain required number of validation instructions. For example, a step with a high risk level may require at least two validation instructions to be entered by a user, in particular before, during and/or after the step. A step may require staggered instructions concerning on the one hand broader aspects and on the other hand more specified aspects of the step.

A step with a medium hight risk level may need only one validation instruction. A step with a low risk level may need no validation at all.

A validation instruction may be a go/no-go instruction.

The computing unit may be adapted to carry out change instructions only if predefined instruction criteria for the respective risk level of the respective step are met.

The medical procedure may be divided in a plurality of separated steps, wherein the system is adapted to automatically request to receive said user instructions before or after the first step or before or after the last step, particularly only before the first and/or after the last step. In this case, typically, the system is operating in the autonomous mode, for which user input regularly is not necessary during the medical procedure.

The system may be adapted for a medical procedure comprising an intraluminal access, such as an intravascular or a gastrointestinal access.

The system may be adapted for intraluminal procedures, that intervene a body duct or cavity, such as TAVI procedures, bariatric procedures, bronchoalveolar procedures, uretro-vesical procedures or vagino-uteral procedures,
In particular, the system may be adapted for a TAVI (transcatheter aortic valve implantation) procedure. The steps of the medical TAVI procedure may comprise at least one of a preoperative step, a planning step, a robot motion step, a puncture step, a navigation step, an intermediate step, a valve placement step and a closing step.

The preoperative step may comprise imaging, in particular a 3D reconstruction. The preoperative step may include a simulation of at least some steps of the TAVI procedure, preferably of the entire TAVI procedure. The preoperative step may include determining critical phases of the procedure and critical areas within the patient. The user may define a respective risk level associated with the step and/or with the patient's area or the computer unit may assess the phases of the procedure and the areas of the patient. The preoperative step may include setting attention marks with respect to respective critical phases of the procedure or with respect to respective critical areas of the patient. The attention marks may be provided during subsequent steps of the medical procedure.

The preoperative step may comprise dilating calcified vessels or dilating the native valve, in particular with a separate instrument.

The planning step may comprise at least one of (i) defining a puncture location and/or an access point, (ii) defining an access path, (iii) defining at least one of a implant valve type, an implant position within the aortic valve and an implant valve size.

Usually, a combination of the type of the implant valve having a suitable size, its intended position within the native aortic valve, a suitable access point and an appropriate path leading from the access point to the intended position in the aortic valve has to be specified. Each of the planning steps may either be entered by the user, may be chosen by the user from a list stored in the system, may be defined by the computing unit and/or may be changed by the user.

The robot motion step may comprise at least one of a detection of an access patch and/or an access point and moving the surgical robot, in particular a surgical tool mounted thereon, to the access point or an access patch.

The robot motion step may be guided automatically be the computing unit. The user may change the access point and the position and orientation of the surgical robot at any time before and during the robot motion step.

The puncture step may include at least one of detecting an artery, targeting a needle, placing the needle, i.e. puncturing, placing an introducer and placing a dilator. The targeting of the needle may for example be bifurcation based.

Before moving the needle inside the patient, the system may position and re-orient the needle tip according to a target point inside the patient, e.g. the artery bifurcation.

As the needle may be positioned and oriented outside the human body access may be performed by only moving the needle along the longitudinal direction of its shaft.

The navigation step may comprise moving an implant valve from an access point to a target location. The navigation step may comprise placing a guidewire, introducing the valve in the artery, detecting specific anatomical characteristics, such as thin vascular walls, vascular branches, vascular bulges or calcifications, determining parameters for the movement of the valve according to the anatomical characteristics, for example slowing the movement within the aortic arch or choosing a specific tool for advancing, for example a balloon for vascular dilatation, if necessary, and moving the valve along the guidewire according to the parameters.

Before or during each of the navigation steps the computing unit may request validation instructions before proceeding.

The navigation step may comprise dilating calcified vessels or dilating the native valve.

The intermediate step may comprise crossing the native valve with the implant valve. This step may need an additional validation instruction in addition to other validations.

The valve placement step may comprise moving the implant valve within the native valve, keeping the implant valve at a chosen position, deploying the implant valve and checking the functionality of the deployed implant valve.

During moving of the implant valve the path may be changed by the operator or based on a proposal of the computing unit.

The deployment of the implant valve may comprise the release and the unfolding of the implant valve. The pressure of a deployment balloon may be adapted during the deployment step.

The functionality of the deployed implant valve may be tested by detecting a pressure gradient along the implant valve or by imaging the valve in the presence of a contrast agent. The quantity of the contrast agent may be adjusted during the step.

An output device may emit acoustic and/or visual signals, such as beeps, voice signals or LED signals, during the navigation to assist a user guided localisation, in particular, during artery access, valve crossing or placement.

Each step or at least one step of the valve placement steps may be guided by the user, may be controlled by the computing unit and/or may be changed by the user.

A validation instruction entered by an operator may be requested to ensure that the implant valve is correctly placed and anchored.

The closure step may comprise the retrieval of the surgical tools, the closure of the artery and a check if the artery is properly closed, for example via ultrasound imaging or via fluoroscopy.

Each step or at least one step of the closure steps may be guided by the user, may be controlled by the computing unit and/or may be adapted by the user.

The system may also be adapted for a transcatheter valve implantation procedure, in particular of heart valves other than the aortic valve. In this case the steps of the medical procedure may comprise the same or similar steps as for the TAVI procedure, which has been adopted to the specific valve.

Alternatively, the system may be adapted for bariatric procedures. The steps of the medical procedures may then comprise at least one of a bariatric preoperative planning step, a bariatric navigation step, a bariatric volume measurement step, a bariatric stomach reduction step, a bariatric safety check step, a bariatric volume verification step, a bariatric implant landing step and a bariatric retraction step. Each bariatric procedure step may comprise sub steps.

Bariatric surgery generally refers to surgical procedures with an attempt to reduce the weight of severely overweight patients. Procedures in such a context include sleeve gastrectomy and/or use implants such as a gastric bypass or gastric band. Within this application bariatric procedures in particular may refer to trans-oral placement of an endoscopic gastrointestinal bypass device, of an intragastric balloon, of a gastric stimulator, of a duodenal sleeve or on an anastomosis device. The bypass device may be a cuff to be attached to the distal oesophagus by transmural anchors and to be connected to a 120cm sleeve diverting undigested nutrients to the jejunum.

A bariatric procedure may also refer a "sleeve gastroplasty", wherein the volume of the stomach is reduced endoscopically by application of sutures, staples or anchors.

In a bariatric volume measurement step first the total volume may be detected. In a bariatric stomach reduction step sutures, staples or anchors may be applied.

In a bariatric safety check step the suture depth, the suture location and/or the suture strength may be detected and may be compared to at least one predetermined specification. It may be determined if the suture depth, the suture location and/or the suture strength comply with the at least one predetermined criterion.

In a bariatric volume verification step it may be detected, if the intended final volume has been achieved.

The bariatric preoperative planning step, preferably using a virtual "standard" patient, may comprise defining a stomach volume reduction, the kind of bariatric treatment, an implant size and in particular a length, further bariatric procedure steps, a navigation path, checkpoints, a deployment site or an initial position of the surgical robot.

Via the user interface the kind of bariatric treatment can be chosen, for example the operator can plan a gastric sleeve implantation or a stomach volume reduction.

A virtual patient may be created based on patient morphology, which may be determined by an imaging method and/or by preceding measurements.

The bariatric navigation step may comprise moving an implant to a target location, introducing a tool and/or the implant into the gastrointestinal tract, detecting specific anatomical characteristics, setting parameters for the movement of the implant according to the anatomical characteristics, for example slowing the movement during the sphincter crossing, and moving the implant according to the parameters.

The bariatric navigation step may comprise controlling the correct path towards and within the stomach, the crossing of the pharynx, the crossing of the sphincter and/or the reaching of target location.

An output device may emit acoustic and/or visual signals, such as beeps, voice signals or LED signals, during the navigation to assist a user guided localisation.

While crossing the pharynx and/or sphincter, air pressure or a force of a deployment tool may be adapted.

When the implant is crossing the pharynx the computing unit may generate a signal to be sent to the user interface and/or to an output interface, with which the patient is invited to swallow.

The bariatric navigation step may comprise imaging the process of inserting the implant for example in a 3D model reconstruction.

The bariatric navigation step may be performed autonomously.

The bariatric implant landing step may comprise positioning the implant at a final position within the gastrointestinal tract, deploying the implant, in particular anchoring the implant and dilating the implant.

During landing, the operator my adjust the position and the orientation of an implant and may adjust the pressure, e.g. of an inflatable anchor, preferably based on sensors on the device and/or on the delivery system, such as a pressure sensor, force sensor.

The bariatric retraction step may comprise a detachment of the tool.

Each step or at least one step of the bariatric procedure steps may be guided by the user, may be controlled or assisted by the computing unit or may be amended by the user.

After or during each step or single steps a validation instruction may be requested.

The system may comprise an image tool, such as an endoscopic camera, and/or any other measurement device for live measurement of the stomach to monitor all bariatric steps. The image tool may comprise an endoscopic camera, a stereo camera, narrow band imaging device and/or an ultrasound imaging device.

The live measurement may be based on real-time stereo reconstruction or using a laser/light source to project a pattern on the wall of the stomach to help with a stereo 3D reconstruction.

The image tool may show a digestive action before or during the bariatric procedure, for example a path which is taken by food on a live video.

Alternatively, the system may be adapted for bronchoalveolar procedures, analogue to the bariatric procedure as described above. The steps of the medical procedures may then comprise at least one of a bronchoalveolar preoperative planning step, a bronchoalveolar navigation step, a bronchoalveolar volume measurement step, a bronchoalveolar safety check step, a bronchoalveolar volume verification step, a bronchoalveolar implant landing step and a bronchoalveolar retraction step. Each bronchoalveolar procedure step may comprise sub steps.

Alternatively, the system may be adapted for uretro-vesical procedures, analogue to the bariatric procedure as described above. The steps of the medical procedures may then comprise at least one of a uretro-vesical preoperative planning step, a uretro-vesical navigation step, a uretro-vesical volume measurement step, a uretro-vesical safety check step, a uretro-vesical volume verification step, a uretro-vesical implant landing step and a uretro-vesical retraction step. Each uretro-vesical procedure step may comprise sub steps.

Alternatively, the system may be adapted for vagino-uteral procedures, analogue to the bariatric procedure as described above. The steps of the medical procedures may then comprise at least one of a vagino-uteral preoperative planning step, a vagino-uteral navigation step, a vagino-uteral volume measurement step, a vagino-uteral safety check step, a vagino-uteral volume verification step, a vagino-uteral implant landing step and a vagino-uteral retraction step. Each vagino-uteral procedure step may comprise sub steps.

The computing unit may be adapted to determine a risk parameter associated with changes based on change instructions entered via the change interface. The computing unit may further be adapted to provide the risk parameter or to request a validation instruction, for example a further change instruction or a go/no-go instruction, depending on the risk parameter. The computing unit may further be adapted to provide a warning signal, preferably an optical or acoustical warning signal, depending on the risk parameter. In any a case respective signal is sent to an output interface.

The risk parameter may be one selected from predetermined risk categories, for example predetermined risk levels, and/or the risk parameter may be associated with a series of consequences due to the change instruction, such as failure of the medical procedure step or harm to the patient.

The system may comprise at least one separate emergency stop device. The separate emergency stop device may be arranged close to patient and/or close to the computing unit and/or close to the surgical robot, such that a user may react immediately on an unexpected event.

The separate emergency stop device may be integrated in the user interface, for example as a separate button or control area, for example displayed on a monitor. Preferably, the separate emergency stop device is provided as a separate physical unit.

Preferably, the separate emergency stop device is provided in addition to the stop interface.

The system may comprise an output interface for providing information to the user, in particular relating to at least one of said steps.

The output interface may be adapted to receive signals from the computing unit and preferably to receive signals from units separate from the system, such as sensor devices.

The output interface may comprise a visual and/or an acoustical unit. The visual unit may display light signals, text, images, films and/or hologram information. The output unit may be part of the user interface.

The output interface may be selected from the group of optical or visual output interfaces, an augmented reality display, a virtual reality display, a haptic feedback device or acoustic output interfaces.

The optical or visual output interfaces may comprise a light emitter, such as a LED lights or a monitor, or a projector, in particular for projecting information, for example a picture or a film, on the patient's body or for providing a 3D monitor, for example a holographic projector. The optical or visual output interfaces may comprise VR-goggles.

The output interface may be a combination of a 3D monitor and a haptic feedback device, such as a haptic glove.

The output interface may be able to receive user input and may relate this input into to the displayed information. For example, the output device may be combination of a hologram display device and haptic glove, such that the user may rotate and shift the hologram.

The output interface may support the user in making his/her input. For example, the user may change the path by moving the animated path presented by the output interface.

The acoustic output interfaces may provide an acoustic alarm signal or may comprise a speech synthesiser.

The output interface may be part of the user interface.

The computing unit may be adapted to generate signals and to send them to the output interface.

The computing unit may be adapted to provide information to the output interface selected from the group of
- information on the current step carried out by the system and/or on the next step to be carried out by the system,
- a checklist comprising, preferably all, steps of the intervention,
- information about a digital twin,
- information about a simulated procedure,
- information about landmarks on a patient,
- information about a target area for a needle,
- information about a planned and/or an actual trajectory of an implant,
- information about a passage of an implant at predefined areas,
- information about critical areas and/or critical steps,
- information about a planned and/or an actual instrument retraction,
- information about physiological parameters of the patient and/or environmental data,
- information about a requested input and/or action.

Information on the current step carried out by the system and/or on the next step to be carried out by the system may provide feedback to the operator and/or to the patient about the stage of the procedure.

For example, the computing unit may be adapted to provide information to the output interface about a tool or an implant reaching a region of interest, for example the annulus or the sphincter, indicating that the system is ready for a next procedural step. The information on the current and/or next step may imply a request for one of the precited control instructions.

The computing unit may be adapted to provide information on past or planned steps of the medical procedure to create a report and/or to predict possible next steps in order to facilitate the procedure planning.

The computing unit may create the report or may assist in creating the report.

Information on the current step and/or on the next step can be provided using a detailed checklist which may be updated either automatically in the manual mode, or manually in a degraded mode.

Independently of the operating mode, the computing unit may be adapted to provide information in the form of a checklist comprising, preferably all, steps of the intervention to the output interface. The system may display the checklist.

The checklist may be editable during planning.

Generally, the checklist keeps track of instruments and their position.

In the autonomous mode, a checklist may inform a practician as to which over-arching (interventional) step is performed (e.g., access), a step that is validated, and within this step, which sub-steps are performed and are to be performed (e.g., needle puncture, dilator in, introducer in, dilator out, needle out, etc.).

In degraded modes, a checklist may remind a clinician, which instruments have to be deployed and which manipulations have to be performed. Additionally, the checklist may provide for keeping track of what already happened.

Manual update and/or editing can be done via an input interface for example via a user interface, via a change interface or a via a validation interface as described above.

The virtual twin may comprise a virtual representation of the patient's anatomy of interest, which helps on planning and on intra-operative decision-making. The virtual twin may be shown as a two-dimensional image or as a reconstructed 3d model. The virtual twin may include specific anatomical characteristics of the patient, for example calcifications. The virtual twin may include specific landmarks chosen by the user or determined and set by the computing unit.

The simulated procedure may include information with respect to critical steps of the medical procedure and to critical areas of the patient, which need higher attention by the operator, or the simulated procedure may indicate critical phases and critical areas.

The computing unit may provide data of landmarks on a patient image, in particular on an ultrasonic image. Landmarks may help to merge images of different sources and/or to plan further steps.

The computing unit may propose a target area for a needle insertion, based on a patch set by the user and/or based on the patient's anatomy and/or based on the steps of the medical procedure.

The trajectory of an implant describes the path taken by the implant between an access site and a target site. The trajectory may be presented on a patient image, for example on a virtual twin. The trajectory may be visualized from different points of view, for example from outside or from the tip of the instrument. The trajectory may be visualized in a two- or three-dimensional representation.

Information concerning a passage of an implant at predefined areas may include a detailed representation of the predetermined area and may allow to plan and/or to monitor a procedure step at this area, for example a valve crossing.

Critical steps of the medical procedure and critical areas of the patient may be related to a higher risk level and may need a higher level of care. Critical areas may comprise specific patient's characteristics, such as vascular calcifications, thin vascular walls or vascular bulges. Critical procedure steps may include difficult manoeuvres and/or a complex tool handling.

Parameters of the patient may include measured physiological parameters, such as blood pressure, temperature, flow profiles or pulse oxygen saturation. The parameters of the patient may include live measurements of the stomach. Parameters may be determined by the computing unit due to information provided by a sensor.

Environmental data may comprise data measured outside the patient, such as temperature, ventilation parameters or treatment parameters, such as balloon pressure.

Information about critical events may include warning signals which are for example displayed in case of an unplanned event, such as a deteriorated vital sign of the patient, a system failure or unexpected motion of the patient.

The respective information may be calculated by the computing unit and/or may be taken from stored data and/or may be entered by an operator.

Information about a requested input and/or action may include an invitation for giving a stop, change or continue instruction, such as simple go/no go input, or a proposal for a change of a level of autonomy.

Information about a requested action may include a proposal for an interaction with the patient, for example a request for the patient to swallow or to breathe.

The computing unit may be adapted to process user instructions, in particular to change instructions and to compute a possible result, for example to predict further procedure steps and/or implications on the further procedure steps, and to assess the respective instructions and/or the respective results. The computing unit may be adapted to send the computed outcome to the output interface, in particular to show to the operator which risks are associated with the respective instruction.

The output interface may provide an animated 2D or 3D presentation, preferably from different points of view, which gives the user the possibility to interact, for example for setting landmarks for highlighting regions of interest.

In the animated 2D or 3D presentation, the path and/or the advancing tool and/or the tool in action can be displayed in relation with the image of the patient.

The computing unit and/or the output interface may combine information from different sources and may for example overlay computed or entered data and an ultrasound or X-ray image.

The computing unit may be adapted to change the level of autonomy, in particular to autonomously switch to a lower level of autonomy and/or to propose a change to a higher level of autonomy.

The system may comprise at least one medical tool in operative connection with the actuator. The tool in particular a is selected from the group of
- a puncturing needle,
- a dilator,
- an introducer,
- a guidewire,
- a catheter, preferably with a pre-mounted implant,
- an endoscopic device, in particular a growing robot,
- an imaging device and
- a closing unit.

The dilator may comprise a balloon for dilating vessels, a native valve, for dilating and /or shaping an implant.

The closing unit may comprise a suturing device.

The system may comprise at least one actuator for operating a surgical robot. The system may comprise at least one surgical robot.

The system may comprise a mobile robot cart, for carrying the operating unit comprising the user interface and the computer unit, and/or the surgical robot. The computer unit may be adapted to control the mobile robot cart. The mobile robot cart may comprise a separate control unit allowing to drive the mobile robot cart. The mobile cart may follow the operator to the operation room.

The surgical robot, and in particular the mobile cart, may be in an "on"-mode, an "off"-mode or in a "stand-by" mode. In the "stand-by" mode, the surgical robot may be manually moved directly. The computing unit may be adapted to switch between the "on"-mode, the "off"-mode or in the "stand-by" mode.

The invention also provides a method for autonomously carrying out at least one step of a medical procedure comprising at least one step.

The method preferably is performed using a system as described above.

The method comprises the steps of autonomously controlling operation of an actuator by a computing unit for operating a surgical robot to carry out the at least one step of the medical procedure.

The method further comprises receiving control instructions from a user interface by the computing unit in the form of one of change instructions, stop instructions and continue instructions.

The method further comprises inducing a change operation, a slow-down operation, a speed-up operation, a hold operation, a stop operation or a continue operation of the actuator by the computing unit based on the control instructions as explained above.

The medical procedure may be divided into a plurality of separated steps, wherein the method may comprise automatically requesting an input of said control instruction before, during and/or after at least one of the steps.

When the medical procedure is divided into a plurality of separated steps, the computing unit may act in an autonomous mode and fully control the operation of the actuator to carry out at least one step of the medical procedure.

Additionally or alternatively, the computing unit may act in a fully degraded mode. The fully degraded mode may be a manual degraded mode, where the computing unit releases the actuator for at least one step of the medical procedure. The fully degraded mode may be a teleoperated degraded mode, where the computer unit controls the actuator for at least one step of the medical procedure based on teleoperator control instructions provided by a user.

Additionally or alternatively, the computing unit may act in a partially degraded mode, where the computing unit controls the operation of the actuator to carry out at least one step of the medical procedure based on communication with the user.

As described above, the partially degraded mode may comprise different levels of partially degraded modes, depending on the degree of user influence on the computing unit.

The method may comprise switching between the autonomous mode, the fully degraded mode and/or the partially degraded modes.

There may also be a switching between different levels of the partially degraded modes.

The computer unit may automatically switch from the autonomous mode to the partially degraded mode and/or to the fully degraded mode or from the partially degraded mode to the fully degraded mode. Within this context automatically means, that in the respective mode, the computer unit verifies if conditions for continued operating in the respective mode are fulfilled. If not, the computer unit switches to a less autonomous mode on its own.

The method may comprise switching between modes according to a predetermined hierarchical model.

In particular, the computer may only switch to the next nearest autonomy level. The computer unit may automatically switch to a less autonomous mode and the computer unit may need a control instruction from a user for switching to a more autonomous mode.

In particular, passing from one level to another may only be done autonomously from the fully autonomous level to a semi-autonomous level, after an interventional step has been carried out. The system awaits then human input to validate and initiate the next interventional step. Passing from the semi-autonomous level back to the fully autonomous level can only be done after human input.

Passings from all other levels to a neighbouring level preferably are only done with human input.

If there is no emergency, the computer may switch to the nearest level with lower autonomy and await input/instructions. Preferably, the computer cannot switch to a higher autonomy level on its own.

Preferably, a human user can activate the emergency mode at any time and in any mode.

For safety reasons, the system may be able to detect and signal abnormalities which constitute emergencies, in which case it may pause and await instructions or direct human intervention.

The computer unit may request a control instruction from a user before switching to the autonomous mode or before switching from the fully degraded mode to the partially degraded mode. Thus, preferably the computer unit only changes to a more autonomous mode, if allowed by a user.

The computing unit may receive control instructions concerning at least one of an access point for entering the patient's body, a position of the surgical robot with respect to an access point for entering the patient's body, an implant position, speed and trajectory of an implant insertion, position and/or orientation of an implant, deployment parameters for deploying an implant, in particular balloon pressure for expanding an implant, orientation and speed of instruments during instrument retraction and operation of an imaging device, in particular an endoscopic camera, a checklist and/or a to-do list comprising steps of an intervention.

The control instructions may comprise detailed information and may directly be used to control the actuator. The control instructions may also induce the computer unit to determine respective data for controlling the actuator. The user may either specify the access point, the position of the surgical robot, the implant position etc. or the computer unit is adapted to determine and/or to change the access point, the position of the surgical robot, the implant position etc.

The method may be adapted for an intraluminal procedure.

Generally, the computer unit may plan and/or execute at least one step of the medical procedure. For planning and executing steps the computer unit may use AI software.

The computing unit may receive data, for example concerning the patient's anatomy and state, the technical options and the environmental condition, may determine parameters for controlling steps of the medical procedure, in particular based on the data, and may control the surgical robot based on the determined parameters. The computing unit may provide interim results, may report the ongoing steps to a user and may ask for confirmation.

The method may be adapted for an intraluminal procedures, that intervene a body duct or cavity, such as TAVI procedures, bariatric procedures, bronchoalveolar procedures, uretro-vesical procedures or vagino-uteral procedures.

The computing unit may control at least one of a preoperative step, a planning step, a robot motion step, a navigation step for moving a valve from an access point to a target location, an intermediate step, a valve placement step and a closure step, as explained above.

The method may also be adapted for a bariatric procedure and the computing unit may control at least one of a bariatric preoperative planning step, a bariatric robot positioning step, a bariatric navigation step, a bariatric implant landing step, a bariatric implant deployment step and a bariatric retraction step as explained above.

The method may also be adapted for a bronchoalveolar procedure and the computing unit may control at least one of a bronchoalveolar preoperative planning step, a bronchoalveolar robot positioning step, a bronchoalveolar navigation step, a bronchoalveolar implant landing step, a bronchoalveolar implant deployment step and a bronchoalveolar retraction step similar to the bariatric procedure as explained above.

The method may also be adapted for a uretro-vesical procedure and the computing unit may control at least one of a uretro-vesical preoperative planning step, a uretro-vesical robot positioning step, a uretro-vesical navigation step, a uretro-vesical implant landing step, a uretro-vesical implant deployment step and a uretro-vesical retraction step similar to the bariatric procedure as explained above.

The method may also be adapted for a vagino-uteral procedure and the computing unit may control at least one of a vagino-uteral preoperative planning step, a vagino-uteral robot positioning step, a vagino-uteral navigation step, a vagino-uteral implant landing step, a vagino-uteral implant deployment step and a retraction step similar to the bariatric procedure as explained above.

The computing unit may determine a risk parameter associated with changes based on change instructions entered via the change interface. The computing unit may compare the risk parameter with a predefined risk category, for example a risk level, and may react depending on the comparison. If for example the risk is too high, the computing unit may ask for confirmation and/or for a new control instruction and/or may stop operating the actuator and/or may switch to a lower autonomous level.

The computing unit may receive user input and/or user alterations, may compute next steps based on the user input and may send the outcome of the computation to the output interface. The computing unit may also assess a risk which is related to the outcome and the output device may show the outcome with the associated risks.

The computing unit may provide information to an output interface selected from the group of information on the current step carried out by the system and/or on the next step to be carried out by the system, information on a checklist and/or a to-do list, information about a digital twin, information about a simulated procedure, information about landmarks on a patient image, information about a target area for a needle, information about a planned and/or an actual trajectory of an implant, information about a passage of an implant at predefined areas, information about critical areas, information about planned or actual instrument retraction and information about physiological and/or pathological parameters of the patient and/or environmental data, information about a requested input and/or action, as explained above.

The information provided to the output interface may inform the user about all actions performed by the robot, preferably in a live view, about all actions which will be performed following the present step and/or about all steps that have been performed.

The output unit may provide a final report about the medical procedure, may indicate which steps had which level of autonomy and/or may provide an assessment of the executed steps.

The computing unit may control at least one medical tool in operative connection with the actuator, in particular selected from a puncturing needle, a dilator, an introducer, a guidewire, a catheter, preferably with a pre-mounted implant, an imaging device, an endoscopic device, in particular a growing robot and a closing unit. Growing robots, for example as disclosed in EP 4 066 724 A1, use robot body expansion to move and interact with the environment.

The invention also provides a computer program comprising program code for carrying out the steps of the method as described above when the program is executed on a computer, the program code preferably uses a pre-trained neural network and/or an artificial intelligence software.

The invention also provides a computer program product which can be loaded directly into the internal memory of a digital computer and which comprises software code portions executing the method steps as described above when the program is running on a computer, wherein preferably the software code is adapted to access a pre-trained neural network and/or an artificial intelligence software.

The invention will be better understood with reference to the following description of preferred embodiments and the accompanying drawings, in which:
- Fig. 1: is a schematic view of a first embodiment of the system according to the invention;
- Fig. 2: is a schematic view of a second embodiment of the system according to the invention;
- Fig. 3: is a schematic diagram showing steps of a TAVI procedure;
- Fig. 4: is a schematic flowchart showing substeps of a robot motion step and a puncture step;
- Fig. 5: is a schematic flowchart showing detailed sub steps of a robot motion step;
- Fig. 6: schematically shows a hierarchical model for different modes.

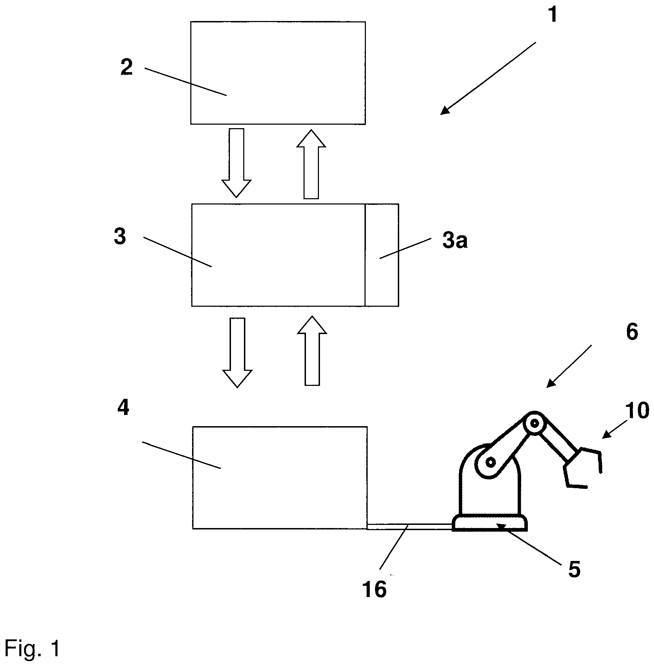

Figure 1 is a schematic view of a first embodiment a system 1 for autonomously carrying out a medical procedure comprising at least one step. The system 1 comprises a user interface 2 for entering user instructions, a computing unit 3 and an interface 4 for communication with at least one actuator 5 for operating a surgical robot 6. The computing unit 3 is adapted to autonomously control operation of the actuator 5 to carry out the at least one step of the medical procedure.

The computing unit 3 is adapted to receive control instructions from the user interface 2 in the form of change instructions, stop instructions and continue instructions to induce a change operation, a stop operation or a continue operation of the actuator 5 based on the control instructions.

The actuator 5 allows to operate different medical tools 10 associated with the surgical robot 6. The interface 4 cooperates with the actuator 5 via a wire connection 16 to send control signals to the surgical robot 6 and to receive signals which may be processed by the computing unit 3. For example, the computing unit 3 may generate a signal to be displayed on a display of the user interface 2.

The system 1 comprises a memory 3a, which allows to store information concerning the procedure steps, concerning risk levels related with the procedure steps and concerning the patient, for example data related to the patient's anatomy.

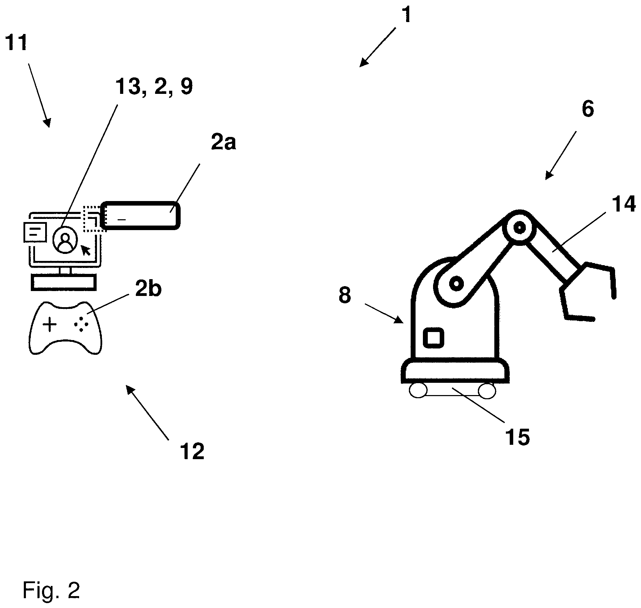

Figure 2 is a schematic view of a second embodiment of the system 1.

The system comprises an operating unit 12 with a computer 11. The computer 11 comprises a computing unit not explicitly shown and a monitor 13, which provides a user interface 2 and an output interface 9.

The computer 11 comprises a validation interface 2a with a gaze tracker and a change interface 2b, in this case a teleoperation console comprising a joystick, buttons and hand motion trackers.

The system 1 further comprises a surgical robot 6, having a robot arm 14, which communicates with the computer 11. The surgical robot 6 comprises an emergency stop device 8 for immediately stopping any surgical procedure when an emergency stop button is activated.

The surgical robot 6 comprises a mobile robot cart 15 which autonomously moves the surgical robot 6 to an operational site.

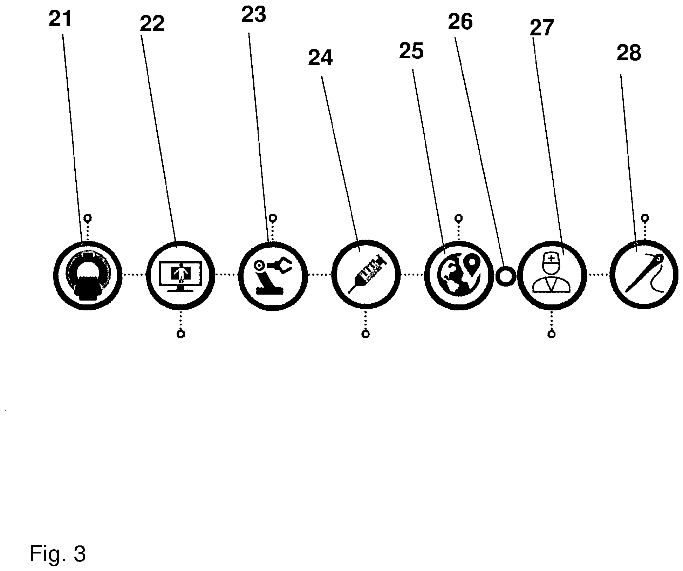

Figure 3 is a schematic diagram showing steps of a TAVI procedure. The TAVI procedure comprises a preoperative step 21, a planning step 22, a robot motion step 23, an access step 24, a navigation step 25, an intermediate step 26, a valve placement step 27 and a closure step 28.

During the preoperative step 21 a three-dimensional image of the relevant vascular system is reconstructed, based on an imaging process, for example a computer tomography with contrast agent and/or a vascular mapping is generated.

A digital twin of the patient may be constructed. The digital twin may be displayed.

During the planning step 22 a puncture location and/or an access point is determined. The computing unit proposes a pathway through the vascular system, an implant valve type, an implant valve size and a specific implant valve position. The operator may validate the respective proposal or may change the selection. The computing unit may check the consequences of the manual selection. Subsequently the computing unit may validate the selection or may return a warning or an alternative proposal.

During the robot motion step 23 the surgical robot 6 and in particular the robot arm 14 (see figure 2) is moved to a place and into an orientation to perform the surgical procedure, for example as disclosed in EP22315330.5.

For bringing the surgical robot 6 to the operation place an access patch may be detected and the mobile robot car 15 may place the surgical robot 6 such that the robot arm 14 may reach the access patch.

During the access step 24 an artery is detected, an appropriate needle is chosen, the needle is placed, and the patient is punctured. After that an introducer is placed at the access point, if necessary, with help of a dilator.

Once for example the femoral bifurcation of the artery is detected, the needle target may be approximately 1-2cm before the bifurcation, ideally with the least amount of calcifications.

Actions on the patient are performed by the robot arm 14, under control of the computing unit 3. The computing unit 3 may plan and perform all substeps of the puncture step autonomously, guided by the user or validated by the user.

During the navigation step 25 a guide wire is laid through the vascular system from the access point into the heart. The implant valve is guided through an introducer and is guided from the access point to the heart along the guid wire. If needed, additional tools are used. For example, if calcification structures have been detected, a balloon may be used to dilate the blood vessel.

The computing unit 3 may plan and perform all substeps of the navigation step 25 autonomously, guided by the user or validated by the user.

At specific anatomical structures, the user may fully take over control or the user influence may be increased. For example, when passing the aortic arch, control instructions may be requested at additional check points. The speed of the movement may be lowered to give the user further opportunities to intervene.

During the intermediate step 26 the valve implant crosses the native valve. This step usually needs a high attention of the operator and can be controlled in a low level of autonomy of the computing unit 3, for example in a fully or partial degraded mode.

It may be necessary to dilate a calcified native valve.

During the valve placement step 27 the valve implant is transferred to its functional state. The valve implant is placed appropriately, is deployed from a catheter, is oriented, is dilated, is anchored and/or is partially or fully released from the catheter. By measuring a pressure gradient and/or by monitoring the flow of a contrast agent the functionality of the valve is validated.

If necessary, the valve implant may be recaptured and may be reloaded into the catheter and repositioned or be removed.

The computing unit 3 may plan and perform all substeps of the valve placement step 27 autonomously, guided or validated by the user.

The navigation step 25, the intermediate step 26 and the valve placement step 27 typically are monitored by an imaging system, for example by an X-ray system. Additionally or alternatively, local information detected by the robot arm may be transferred into a signal to be presented on an output device, for example on a 3d-image of the patient and/or of the digital twin and/or on a virtual reality monitor.

During the closure step 28 all instruments are retrieved from the vascular system. The artery is closed, preferably using a closure device. The closure is validated, for example using fluoroscopy and/or ultrasound.

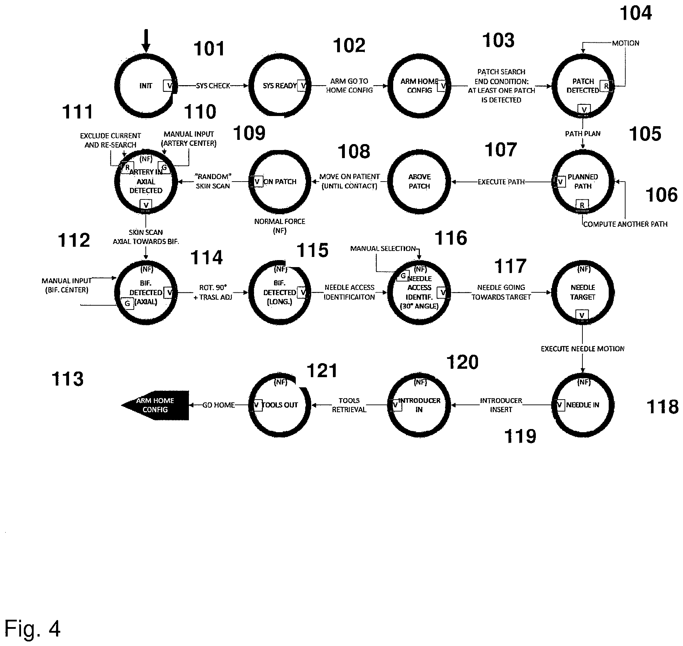

Figure 4 is a schematic flowchart showing substeps of the robot motion step 23 and an access step 24. The substeps may start when the surgical robot has taken its intended position.

In step 101 the system checks if the system is in a ready state. The user may validate the ready state.

In step 102 the robot arm is brought into a home configuration. The user may validate the home configuration.

In step 103 a patch is searched according to a patch search end condition which for example defines, that at least one patch has to be detected, for example as disclosed in EP22315330.5.

For searching the patch a motion step 104 can be repeated wherein a robot arm with a patch detection unit is moved several times until at least one patch is detected.

As soon as the patch has been detected, a path for moving the access tools to the access point is defined in a step 105. Until the final path has been determined, other paths may be planned in a step 106, for example as long as the final path has not been validated by a user.

In a step 107, the path is executed, that means that the tools are positioned above the patch.

In a step 108 the tools are moved towards the patient until there is contact with the patch.

In step 109 a search is performed by an ultrasound probe to scan the skin until the axial position of the artery is detected.

In step 110 the center of the artery may be entered manually.

Alternatively or additionally, in step 111 the search may be repeated.

In step 112 the skin is scanned along the axis of an artery towards a bifurcation until the bifurcation is detected. If needed, in a step 113, the center of bifurcation is entered manually.

In step 114 a rotation of the ultrasound probe by 90° around the US probe shaft takes place so to pass from an axial view of the vessel to a longitudinal one until the bifurcation is longitudinally detected.

In step 115 the needle access is identified. The needle access may be identified as a function of the femoral bifurcation position and/or the femoral head position.

The puncture can be based only on the position of the femoral bifurcation, or it can be based on the position of the femoral head, or a combination of both is possible for more algorithmic robustness and patient safety.

Bifurcation or femoral head position can be identified purely by means of image processing algorithms, purely by means of deep neural networks, or by combining both approaches.

An example of a purely image processing algorithm for the detection of the femoral bifurcation may comprise the following steps.

Firstly, the femoral artery around a femoral bifurcation region is segmented, for example using an active contour method.

Secondly, the centerline (or the skeleton) of the femoral artery is extracted, for example by using mathematical morphology, and the inner contour of the femoral artery is identified using mathematical rules regarding the position of the inner contour with respect to the centerline of the femoral artery.

The inner contour of the femoral artery can then be approximately modelled as an inclined Y-shape, in such a way that the femoral bifurcation can directly be extracted as the bifurcation point of a Y-shape, for example, using mathematical morphological rules.

A combination of deep neural networks with image processing for the detection of the femoral bifurcation can, for example, comprise segmenting the femoral artery around the femoral bifurcation region using a deep neural network (e.g. a 2D Unet).

After that the femoral bifurcation can be extracted by the aforementioned subsequent image processing steps.

A purely deep neural network based on femoral artery bifurcation detection may comprise training a regression deep neural network on labelled images by medical experts in order to directly identify the geometric coordinates of the bifurcation point in an ultrasound image.

An image processing algorithm for femoral head detection in an ultrasound image can comprise detecting the zone of the femoral head with highlighted texture using, for example, adaptive thresholding or a clustering algorithm (e.g.; K-means) in pixel neighborhoods, as well as the prior knowledge of femoral head shapes (e.g.; roundness) and relative position with respect to the artery or/and femoral bifurcation.

A neural network based detection of the femoral head may comprise training a segmentation deep neural network (e.g.; a 2D Unet) trained on a significantly large dataset of labeled images. Alternatively, using prior knowledge about the shape of the femoral head, a parametric shape model (e.g., an ellipse or a higher order shape) may be fitted to the femoral head using a regression deep neural network. The latter will take as an input an ultrasound image and outputs the vector of parameters of the chosen parametric shape model.

If needed, in a step 116 the needle access is identified manually.

For example a bifurcation may be detected as shown in the copending application EP 22315210.9 of the same applicant.

As soon as the needle access is identified, in a step 117 the needle is moved towards the target.

In a subsequent step 118 a needle movement is executed until the needle is inserted.

In a step 119 an introducer is inserted. As soon as the introducer is validated to be properly inserted, the tools may be retrieved in a step 120.

In a final step 121 the puncturing tools may be brought into a home configuration.

During all substeps the operator is in a position to stop, to validate, to edit and /or to intervene at any step. The interaction takes place via a user interface 2, for example in the form of a graphical user interface (GUI), a virtual reality environment, physical buttons, a vocal device, a gaze tracking device, haptic gloves etc.

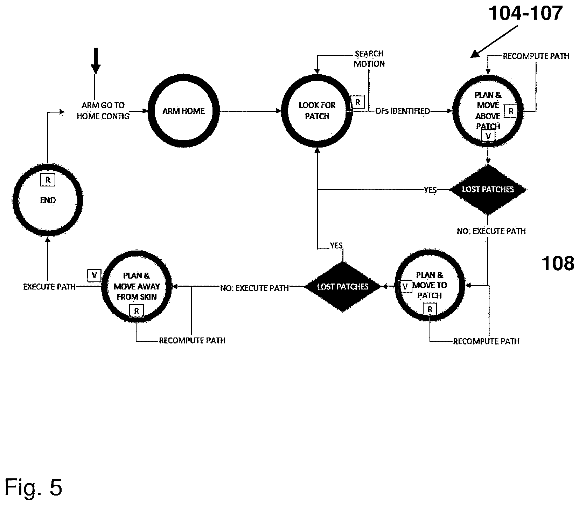

Figure 5 is a schematic flowchart showing further substeps of a robot motion step. The further steps include exit points, in case the next sub step could not be carried out.

For example, after steps 104-107 (see also figure 4) have been performed, and hence a patch has been detected, a path has been planned and the robot arm has been moved above the patch, it may turn out that the patches are lost and cannot not be detected anymore. If patches are lost, steps 104-107 have to be repeated.

If no patches are lost the next step 108 can be performed and the tool can be moved towards the patch.

Again, it may turn out that the patches are lost and cannot not be detected anymore. If patches are lost at this point of time, steps 104-108 have to be repeated. Otherwise, the path may be executed.

If finally no patch can be found anymore the tool has to be moved away from the skin.

The movement away from the skin may comprise in moving in Cartesian space along the direction of the main axis if the Ultrasound probe. This ensures a linear motion of the Ultrasound probe to clear the patient.

As soon as there is contact with the skin of the patient there is no more patch detection module running.

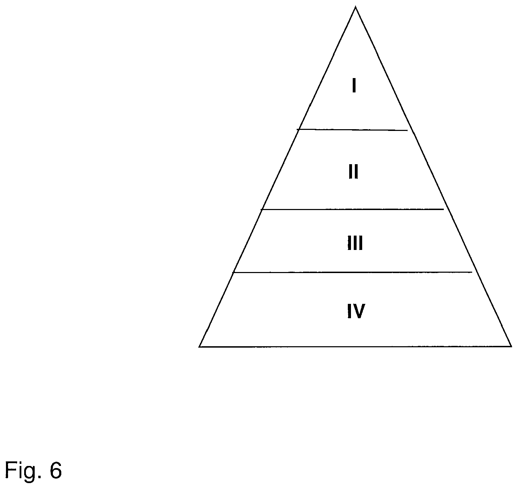

Figure 6 schematically shows a hierarchical model for different modes.

Mode IV corresponds to the lowest level of automatization, wherein the computer unit is only adapted for signal processing under human control.

Mode IV corresponds to level 0 as explained above. The system, for example the system of a surgical robot, does not enact any steps, but some subsystems may keep working, such as signal processing.

This mode can be taken during emergency.

Mode III is related to a degraded autonomy. The user has direct control over the physical devices.

Mode III corresponds to levels 1-2 as explained above. A surgeon either teleoperates or specifies, physically by interacting, placing and/moving the surgical robot to an area of interest, after which the surgical robot can enact a series of steps. Placement may be enabled by overlayed ultrasound images, such as of detected vessels or other anatomical features.

Mode II is related to a semi-autonomous level, wherein an agent is fed by a GUI input.

Mode II corresponds to level 3 as explained above. The surgical robot may have a general plan of what is to be done and/or what is to be achieved or to be detected. Necessary information (e.g., concerning a femoral bifurcation, a suture point) may not be available to the surgical robot or accessible for the surgical robot. The surgeon may provide the required input, for example via a GUI, after which the surgical robot is able to proceed.

Mode I corresponds to a fully autonomous control, under a user's supervision.

Mode I corresponds to level 4 as explained above, and - under the assumption that confirming the robot to continue does not constitute human input/aid - to level 5 as well.

### Reference Numbers

system (1)
user interface (2)
validation interface (2a)
change interface (2b)
stop interface (2c)
computing unit (3)
memory (3a)
(actuator) interface (4)
actuator (5)
surgical robot (6)
switch unit (7)
emergency stop device (8)
output interface (9)
medical tool 10
a computer (11)
operating unit 12
monitor 13
robot arm 14
mobile robot cart 15
wire connection 16
steps (21,...,29; 101,..., 115)
a preoperative step (21)
a planning step (22)
a robot motion step (23)
an access step (24)
a navigation step (25)
an intermediate step (26)
a valve placement step (27)
a closure step (28)

## Claims

1. A system (1) for autonomously carrying out a medical procedure comprising at least one step, the system comprising
- a computing unit (3)
- an interface (4) for communication with at least one actuator (5) for operating at least one surgical robot (6)
- a user interface (2) for entering user instructions
wherein said computing (3) unit is adapted
- to autonomously control operation of the actuator (5) to carry out the at least one step of the medical procedure
- to receive control instructions from said user interface (2) in the form of one of change instructions, stop instructions and continue instructions and
- to induce a change operation, a warning signal, a to-proceed-signal, a stop operation or a continue operation of the actuator based on the control instructions.

2. A system according to claim 1, wherein the medical procedure is divided into a plurality of separated steps (21, ..., 29; 101,..., 115), the system being adapted to automatically request to receive said control instructions before, after and/or during each of the steps (21, ..., 29; 101,..., 115).

3. A system according to claim 1 or 2, wherein the computing unit is operable in an autonomous mode, in a fully degraded mode and/or in partially degraded modes,
wherein
- in the autonomous mode, the computing unit (3) fully controls operation of the actuator (5)
- in the degraded mode, the computing unit (3) (i) releases the actuator (5) so that it can be freely and manually operated by a user in a manual degraded mode or (ii) controls the actuator (5) based on teleoperation control instructions provided by a user in a teleoperated degraded mode.

4. A system according to claim 3, wherein the system comprises a switch unit (7) for switching operation between the autonomous mode, the fully degraded mode and/or the partially degraded modes, and/or in particular between different levels of the partially degraded modes.

5. A system according to claim 4, wherein the switch unit (7) is controlled by the computing unit (3) in such a way that
- operation can be automatically switched from the autonomous mode to the fully or partially degraded mode, and
- operation can only be switched to the autonomous mode upon instruction or validation by a user.

6. A system according to one of the claims 1 to 5, the system (1) comprising a validation interface (2a) for entering validation instructions, the validation interface (2a) preferably being selected from the group of:
- a vocal interface,
- a mechanical button or switch,
- a mouse,
- a joystick,
- a haptic glove,
- a graphical user interface, in particular a touchscreen
- motion detector, in particular a gaze tracker or a head motion detector,
- an eye-to-image interface,
- a virtual reality or an augmented reality interface,
- a 3D monitor,
- a timer.

7. A system according to one of the claims 1 to 6, the system further comprising a change interface (2b) for entering initiate, change, proceed, hold and/or stop instructions, the change interface (2b) preferably being selected from the group of:
- a vocal interface,
- a mechanical button or switch,
- a mouse,
- a joystick,
- a haptic glove,
- a graphical user interface, in particular a touchscreen,
- motion detector, in particular a gaze tracker or a head motion detector,
- an eye-to-image interface,
- a virtual reality or an augmented reality interface,
- a 3D monitor.

8. A system according to claim 7, wherein the change instructions are selected from the group of
- Defining an access point for entering the patient's body
- Defining a position of the surgical robot with respect to an access point for entering the patient's body
- Defining an implant position
- Defining speed and trajectory of an implant insertion
- Defining position and/or orientation of an implant
- Defining deployment parameters for deploying an implant, in particular balloon pressure for expanding an implant
- Defining orientation and speed of instruments during instrument retraction
- operation of an imaging device, in particular an endoscopic camera
- Defining a checklist and/or to-do list.

9. A system according to one of the claims 1 to 8, the system further comprising a stop interface (2c) for providing stop instructions, the stop interface (2c) being selected from the group of:
- a vocal interface,
- a mechanical button or switch,
- a mouse,
- a joystick,
- a haptic glove,
- a graphical user interface, in particular a touchscreen,
- motion detector, in particular a gaze tracker or a head motion detector,
- a virtual reality or an augmented reality interface,
- a 3D monitor,
- a timer,
- a button,
- device for detection of a risk or an unexpected event.

10. A system according to one of the claims 1 to 9, wherein the system (1) has a memory for storing a risk level associated with at least one of said steps (21, ..., 29; 101, ..., 115) .

11. A system according to claim 10, wherein the computing unit (3) is adapted to carry out change instructions only if predefined instruction criteria for the respective risk level are met.

12. A system according to claim 1, wherein the medical procedure is divided in a plurality of separated steps (21, ..., 29; 101, ..., 115), wherein the system is adapted to automatically request to receive said user instructions before or after the first step or before or after the last step.

13. A system according to one of the claims 1 to 12, wherein the system (1) is adapted for intraluminal procedures that intervene a body duct or cavity, such as TAVI procedures, bariatric procedures, bronchoalveolar procedures, uretro-vesical procedures or vagino-uteral procedures, and wherein the steps of the medical procedures comprise at least one of
- a preoperative step (21),
- a planning step (22),
- a robot motion step (23),
- a puncture step (14),
- a navigation step (15) for moving a valve from an access point to a target location,
- an intermediate step (26),
- a valve placement step (27),
- a closure step (28),
- a bariatric preoperative planning step,
- a bariatric robot positioning step,
- a bariatric navigation step,
- a bariatric volume measurement step,
- a bariatric stomach reduction step,
- a bariatric safety check step,
- a bariatric volume verification step,
- a bariatric implant landing step,
- a bariatric implant deployment step,
- a bariatric retraction step,
- a bronchoalveolar preoperative planning step,
- a bronchoalveolar robot positioning step
- a bronchoalveolar navigation step,
- a bronchoalveolar volume measurement step,
- a bronchoalveolar safety check step,
- a bronchoalveolar volume verification step,
- a bronchoalveolar implant landing step,
- a bronchoalveolar implant deployment step,
- a bronchoalveolar retraction step
- a uretro-vesical preoperative planning step,
- a uretro-vesical robot positioning step
- a uretro-vesical navigation step,
- a uretro-vesical volume measurement step,
- a uretro-vesical safety check step,
- a uretro-vesical volume verification step,
- a uretro-vesical implant landing step,
- a uretro-vesical implant deployment step,
- a uretro-vesical retraction step
- a vagino-uteral preoperative planning step,
- a vagino-uteral robot positioning step
- a vagino-uteral navigation step,
- a vagino-uteral volume measurement step,
- a vagino-uteral safety check step,
- a vagino-uteral volume verification step,
- a vagino-uteral implant landing step,
- a vagino-uteral implant deployment step,
- a vagino-uteral retraction step.

14. A system according to one of the claims 7 to 13, the computing unit (3) further being adapted to determine a risk parameter associated with changes based on change instructions entered via the change interface.

15. A system according to one of the claims 1 to 14, the system further comprising a separate emergency stop device (8) .

16. A system according to one of the claims 1 to 15, the system further comprising an output interface (9) for providing information relating to at least one of said steps.

17. A system according to claim 16, wherein the output interface (9) is selected from the group of
- optical or visual output interfaces,
- an augmented reality display,
- a virtual reality display,
- a haptic feedback device,
- acoustic output interfaces.

18. A system according to one of the claims 16 or 17,
wherein the computing unit (3) is adapted to provide information to the output interface (9) selected from the group of:
- information on the current step carried out by the system and/or on the next step to be carried out by the system
- information on a checklist and/or a to-do list,
- information about a digital twin
- information about a simulated procedure
- information about landmarks on a patient image
- information about a target area for a needle,
- information about a planned and/or an actual trajectory of an implant
- information about a passage of an implant at predefined areas
- information about critical areas
- information about a planned or actual instrument retraction
- information about physiological and/or pathological parameters of the patient and/or environmental data,
- information about critical events,
- information about a requested input and/or action.

19. A system according to one of the claims 1 to 18, the system further comprising at least one medical tool (10) in operative connection with the actuator, in particular a tool selected from the group of
- a puncturing needle,
- a dilator,
- an introducer,
- a guidewire,
- a catheter, preferably with a pre-mounted implant,
- an endoscopic device, in particular a growing robot,
and
- a closing unit.

20. A method for autonomously carrying out a medical procedure comprising at least one step, preferably with a system according to at least one of the preceding claims,
the method comprising
- a computing unit (3) autonomously controlling operation of an actuator (5) for operating a surgical robot to carry out the at least one step of the medical procedure,
- the computing unit (3) receiving control instructions from a user interface (2) in the form of one of change instructions, stop instructions and continue instructions and
the computing unit (3) inducing a change operation, a slow-down operation, a speed-up operation, a hold operation, a stop operation or a continue operation of the actuator (5) based on the control instructions.

21. The method according to claim 20, wherein the medical procedure is divided into a plurality of separated steps (21, ...,29; 101, ..., 115), the method comprising automatically requesting said control instruction before, during and/or after at least one of the steps (21, ...,29; 101, ..., 115).

22. The method according to one of claims 20 or 21, wherein the medical procedure is divided into a plurality of separated steps and wherein
- the computing unit (3) acts in an autonomous mode and fully controls the operation of the actuator (5) to carry out at least one step of the medical procedure,
- the computing unit (3) acts in a fully degraded mode, wherein in a manual degraded mode the computer unit (3) releases the actuator (5) for at least one step of the medical procedure and in a teleoperated degraded mode the computer unit (3) controls the actuator (5) for at least one step of the medical procedure based on teleoperator control instructions provided by a user, and/or
- the computing unit (3) acts in a partially degraded mode and controls the operation of the actuator (5) to carry out at least one step of the medical procedure based on a communication with the user.

23. The method according to claim 22, the method comprises switching between the autonomous mode, the fully degraded mode and/or the partially degraded modes, and/or, in particular, between different levels of the partially degraded modes, in particular according to a predetermined hierarchical supervision model.

24. The method according to claim 23, the computer unit (3) automatically switches from the autonomous mode to the partially degraded mode and/or to the fully degraded mode or from the partially degraded mode to the fully degraded mode
and/or
the computer unit (2) requests a control instruction from a user before switching to the autonomous mode or before switching from the fully degraded mode to the partially degraded mode.

25. The method according to one of claims 20 to 24, wherein the computing unit (3) receives control instructions concerning at least one of
- an access point for entering the patient's body,
- a position of the surgical robot with respect to an access point for entering the patient's body,
- an implant position,
- speed and trajectory of an implant insertion,
- position and/or orientation of an implant,
- deployment parameters for deploying an implant, in particular balloon pressure for expanding an implant,
- orientation and speed of instruments during instrument retraction,
- operation of an imaging device, in particular an endoscopic camera,
- a checklist and/or a to-do list comprising steps of an intervention.

26. A method according to one of the claims 20 to 25,
wherein the method is adapted for intraluminal procedures, such as TAVI procedures, bariatric procedures, bronchoalveolar procedures, uretro-vesical procedures or vagino-uteral procedures, and wherein the computing unit (3) controls at least one of
- a preoperative step,
- a planning step,
- a robot motion step,
- a navigation step for moving a valve from an access point to a target location,
- an intermediate step,
- a valve placement step and
- a closure step,
- a bariatric preoperative planning step,
- a bariatric robot positioning step,
- a bariatric navigation step,
- a bariatric implant landing step,
- a bariatric implant deployment step and
- a bariatric retraction step,
- a bronchoalveolar preoperative planning step,
- a bronchoalveolar robot positioning step
- a bronchoalveolar navigation step,
- a bronchoalveolar volume measurement step,
- a bronchoalveolar safety check step,
- a bronchoalveolar volume verification step,
- a bronchoalveolar implant landing step,
- a bronchoalveolar implant deployment step,
- a bronchoalveolar retraction step
- a uretro-vesical preoperative planning step,
- a uretro-vesical robot positioning step
- a uretro-vesical navigation step,
- a uretro-vesical volume measurement step,
- a uretro-vesical safety check step,
- a uretro-vesical volume verification step,
- a uretro-vesical implant landing step,
- a uretro-vesical implant deployment step,
- a uretro-vesical retraction step
- a vagino-uteral preoperative planning step,
- a vagino-uteral robot positioning step
- a vagino-uteral navigation step,
- a vagino-uteral volume measurement step,
- a vagino-uteral safety check step,
- a vagino-uteral volume verification step,
- a vagino-uteral implant landing step,
- a vagino-uteral implant deployment step,
- a vagino-uteral retraction step.

27. A method according to one of the claims 20 to 26,
wherein the computing unit (3) determines a risk parameter associated with changes based on change instructions entered via the change interface.

28. A method according to one of the claims 20 to 27,
wherein the computing unit (3) provides information to an output interface selected from the group of
- information on the current step carried out by the system and/or on the next step to be carried out by the system,
- information on a checklist,
- information about a digital twin,
- information about a simulated procedure,
- information about landmarks on a patient image,
- information about a target area for a needle,
- information about a planned and/or an actual trajectory of an implant,
- information about a passage of an implant at predefined areas,
- information about critical areas,
- information about planned or actual instrument retraction and
- information about physiological parameters of the patient and/or environmental data,
- information about critical events,
- information about a requested input and/or action.

29. A method according to one of the claims 20 to 28,
wherein the computing unit (3) controls at least one medical tool in operative connection with the actuator (5), in particular selected from
- a puncturing needle,
- a dilator,
- an introducer,
- a guidewire,
- a catheter, preferably with a pre-mounted implant,
- an endoscopic device, in particular a growing robot,
- an imaging device and
- a closing unit.

30. A computer program comprising program code for carrying out the steps of the method according to any one of the claims 20 to 29 when the program is executed on a computer (11), the program code preferably using a pre-trained neural network and/or an artificial intelligence software.

31. A computer program product which can be loaded directly into the internal memory of a digital computer (11) and which comprises software code portions executing the method steps of at least one of the claims 20 to 30 when the program is running on a computer (11), wherein preferably the software code is adapted to access a pre-trained neural network and/or an artificial intelligence software.
